# NEW EUROPEAN PATENT SPECIFICATION

(11) **EP 0 269 601 B2**
(45) Date of publication and mention of the opposition decision: **12.07.2000**
(45) Mention of the grant of the patent: 08.09.1993
(21) Application number: 87870161.4
(22) Date of filing: 19.11.1987
(51) Int. Cl.: C12N 15/82, A01H 1/00

(54) **Insect-resistant tomato plants**
Insektresistente Tomatenpflanzen
Plants de tomate résistant aux insectes

(30) Priority: 20.11.1986 US 932818
(43) Date of publication of application: 01.06.1988
(73) Proprietor: Monsanto Company, St. Louis Missouri 63167-7020 (US)
(72) Inventor: Fischhoff, David Allen, Webster Groves Missouri 63119 (US); Rogers, Stephen Gary, Chesterfield Missouri 63017 (US)
(74) Representative: UEXKÜLL & STOLBERG

(56) References cited:
- EP-A- 0 142 924
- EP-A- 0 185 005
- EP-A- 0 193 259
- Nature, 328 (1987), pp. 33-37, Vaeck et al.
- ENGINEERED ORGANISMS IN THE ENVIRONMENT, 1985, pages 40-46, American Society for Microbiology, Washington, US; L.S. WATRUD et al.: "Cloning of the Bacillus thuringiensis subsp. kurstaki delta-endotoxin gene into Pseudomonas fluorescens: Molecular biology and ecology of an engineered microbial pesticide"
- BIOTECHNOLOGY, vol. 5, no. 8, August 1987, pages 807-813, New York, NY, US; D.A. FISCHHOFF et al.: "Insect tolerant transgenic tomato plants"
- NATO ASI SERIES MEETING, Riva del Garda, Italy, 6th-15th October, vol. H-13: "Toxicology of pesticides: experimental, clinical and regulatory perspectives", 1987, pages 241-252, Springer-Verlag, Berlin, DE; D.A. FISCHHOFF: "Role of biotechnology in pesticide development: Bacillus thuringiensis as an example"
- PLANT CELL REPORTS, vol. 5, 1986, pages 81-84, Springer-Verlag; S. McCORMICK et al.: "Leaf disc transformation of cultivated tomato (L. esculentum) using Agrobacterium tumefaciens"
- CHEMICAL ABSTRACTS, vol. 105, 1986, page 270, abstract no. 110476d, Columbus, Ohio, US; D.J. SCHUSTER: "Impact of insecticides on lepidopterous larval control and leafminer parasite emergence on tomato"
- CHEMICAL ABSTRACTS, vol. 75, 1971, page 262, abstract no. 47816j, Columbus, Ohio, US; C.S. CREIGHTON et al.: "Control of caterpillars on tomatoes with chemicals and pathogens"
- Chemical Abstracts, vol. 102 (1985), no. 161301p, Wabiko et al.
- Plant Phys., 85 (1987), pp. 1103-1109, Barton et al.
- Chemical Abstracts, vol. 109, 1988, p. 211, abstract no. 143899e, Columbus, Ohio, US; M.J. Adang et al.

## Description

*Bacillus thuringiensis* is a spore forming soil bacterium which is known for its ability to produce a parasporal crystal protein which is toxic to a wide variety of Lepidopteran larvae. The crystals, which account for about 20-30% of the dry weight of sporulated cultures, consist primarily of a single, high molecular weight protein (∼134 kilodaltons (KD)) which is produced only during sporulation. The crystal protein is produced in the bacterium as the protoxin which is activated in the gut of susceptible larvae to produce a toxic protein having a molecular weight of about 67 KD.

Insects susceptible to the action of the protein toxin of Lepidopteran-type *Bacillus thuringiensis* bacteria include, but are not limited to, tomato pinworm (*Keiferia lycopersicella*), tobacco hornworm (*Manduca sexta*), tomato hornworm (*Manduca quinquemaculata*), beet armyworm (*Spodoptera exigua*), cabbage looper (*Trichoplusia ni*), and tomato fruitworm (*Heliothis zea and Heliothis virescens*). Larvae of these insects are known to feed on tomato plants.

Commercial insecticidal preparations containing spores and crystalline protein produced by *Bacillus thuringiensis* are available under such names as DIPEL® and THURICIDE®. Significant limitations in the use of commercial preparations of the crystalline protein toxin of *B. thuringiensis* include the need for repeated applications of the insecticidal preparations. Another disadvantage is that the crystal protein is only produced during the sporulation stage of the life cycle of *B. thuringiensis.* Such a growth phase limitation, particularly in an industrial process, can result in inconvenience and excessive time requirements during manufacture. At the completion of sporulation, the self-lysing cells release both spores and crystals into the culture medium. Because of environmental concerns it is desirable that commercial insecticidal preparations be substantially free of spores. Unfortunately, because of the similarity in size and density of the spores and crystal protein toxin, separation of the crystals from the spores is complicated and laborious and thus, costly. Further, pressures resulting from growth phase limitations or other factors may result in preparations of *B. thuringiensis* losing their ability to produce the crystals; such acrystalliferous strains do not have insecticidal activity.

The isolation of DNA from *B. thuringiensis* coding for the crystal protein toxin and the insertion of this DNA into expression vectors for the transformation of bacterial species and plant species is known.

EP-A-0 193 259 discloses a chimeric plant gene which comprises :
(i) a promoter functional in plants, being the MAS (PTR2) promoter ;
(ii) a B.t. toxin coding sequence ; and
(iii) a 3'-untranslated sequence containing a functional poly (A) signal.

EP-A-0 142 924 discloses an analogous gene under the control of the "1.6" region of the T-DNA (i.e. the MAS promoter) or the CaMV 35S promoter.

EP-A-0 193 259 and EP-A-0 142 924 furthermore disclose methods to produce genetically transformed Solanaceae plants exhibiting toxicity towards Lepidopterian larvae, transformed plant cells and differentiated plants.

S. Mc Cornick et al., Plant Cell Reports, 5, 81-84, (1986), disclose a method to produce genetically transformed tomato plants via a leaf disc transformation / regeneration system. The disclosed results provided evidence for both single and multicopy insertions of the T-DNA, and have demonstrated inheritance of the T-DNA insert in the expected Mendelian ratios, and have shown that several heterologous promoters function in tomato plants.

However, the prior art does not teach with particularity and enablement how such DNA can be inserted into the genome of a tomato plant such that the toxin will exhibit insecticidal activity against susceptible Lepidopteran larvae.

### BRIEF DESCRIPTION OF THE DRAWINGS

Figure 1 illustrates the coding sequence for a crystal protein toxin of *Bacillus thuringiensis* subspecies *kurstaki* HD-1.
Figure 2 illustrates the preparation of plasmid pMON9733.
Figure 3 illustrates the preparation of plasmid pMAP17.
Figure 4 illustrates the preparation of plasmid pMON294.
Figure 5 illustrates the preparation of plasmid pMON8053.
Figure 6 illustrates the preparation of plasmid pMON9712.
Figure 7 illustrates the preparation of plasmid pMON9741.
Figure 8 illustrates the preparation of plasmid pMON9756.
Figure 9 illustrates the make-up of plasmid pMON316.

### STATEMENT OF THE INVENTION

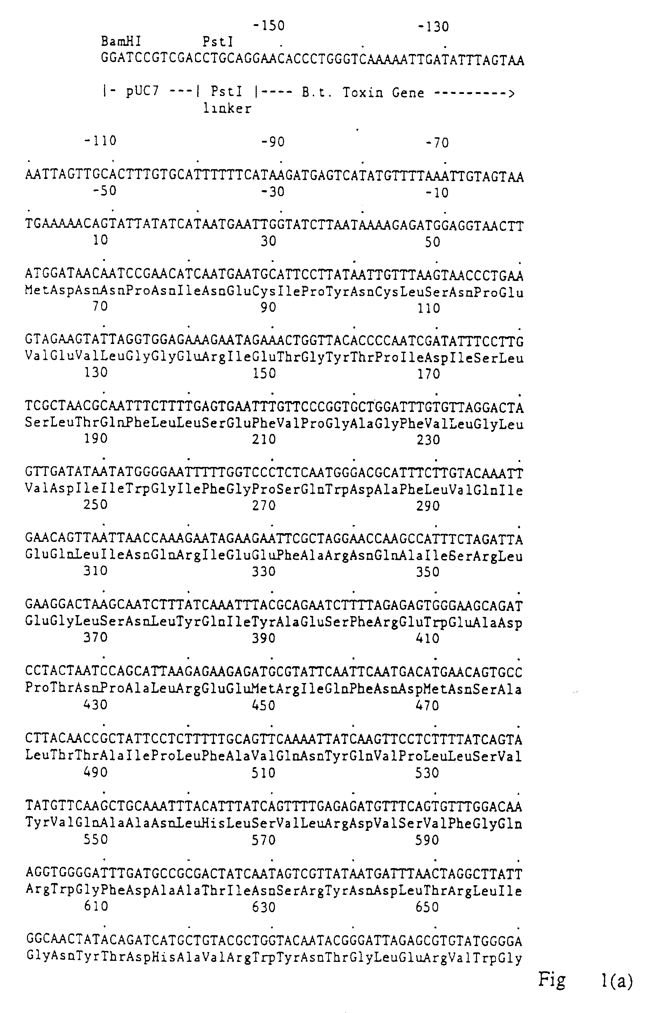

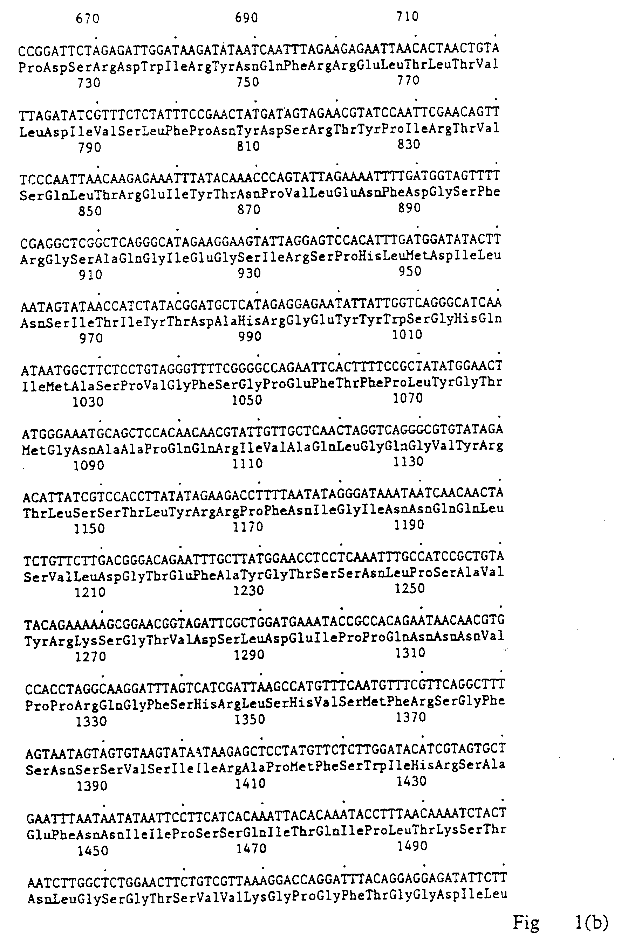

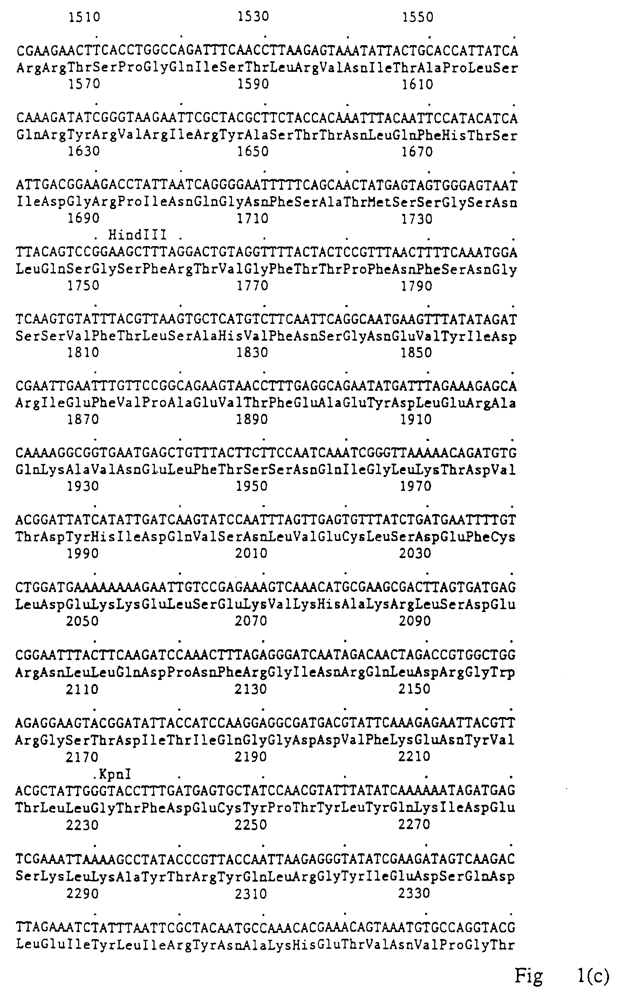

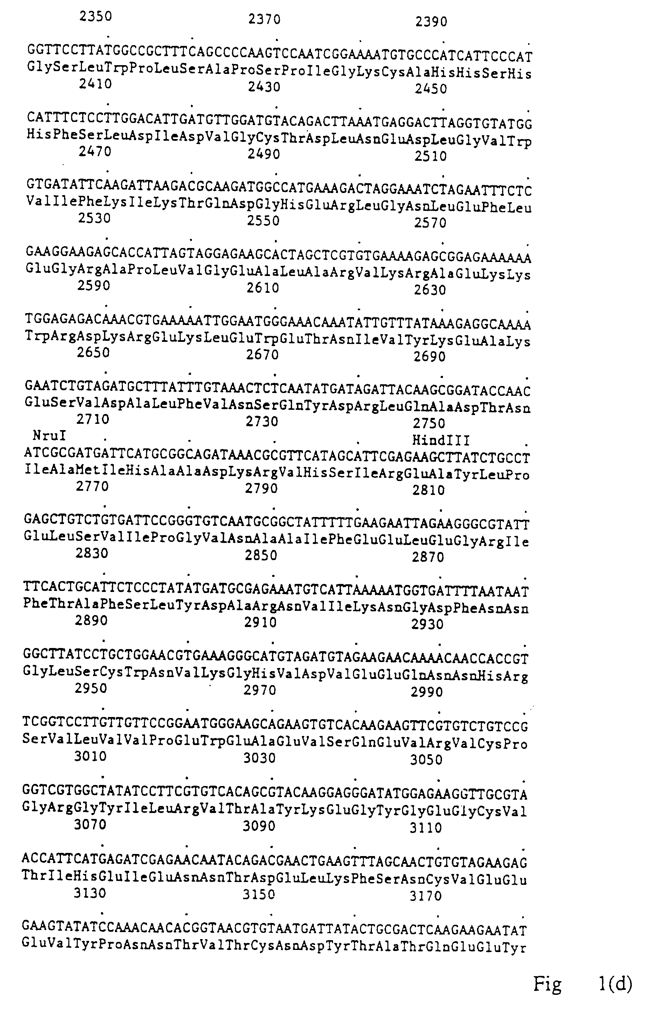

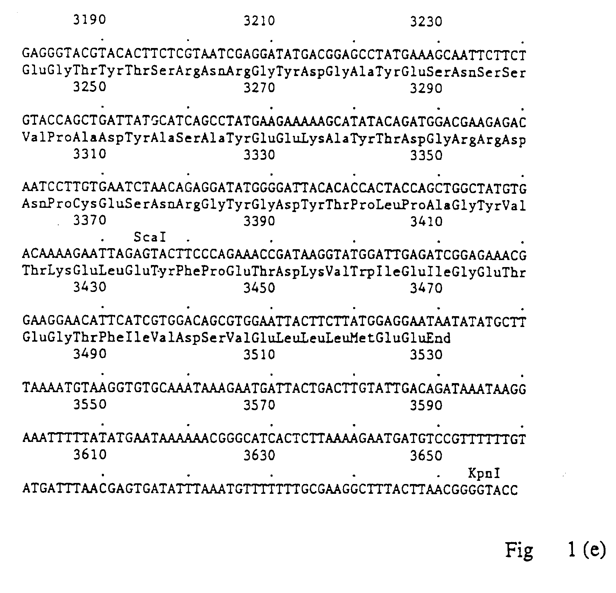

The present invention provides a method of producing genetically transformed tomato plants which exhibit insecticidal activity toward Lepidopteran larvae which comprises:
(a) inserting into the genome of a tomato cell a chimeric gene which comprises
   (i) a promoter which functions in plants to cause the production of a mRNA transcript;
   (ii) a coding sequence that causes the production of mRNA encoding a toxin protein of *Bacillus thuringiensis* said mRNA being the transcript of the DNA sequence 1-3471 of Figure 1, and
   (iii) a 3' non-translated region which functions in tomato to cause the addition of polyadenylate nucleotides to the 3' end of the mRNA.
(b) selecting transformed tomato cells; and
(c) regenerating from the transformed tomato cells genetically transformed tomato plants which exhibit insecticidal activity toward Lepidopteran larvae.

The invention also provides tomato cells and plants thus transformed.

In one aspect, the present invention comprises chimeric genes which function in tomato plants and produce transgenic tomato plants which exhibit insecticidal activity toward susceptible Lepidopteran larvae. The expression of a plant gene which exists as double-stranded DNA form involves the transcription of messenger RNA (mRNA) from one strand of the DNA by RNA polymerase, and processing of the mRNA primary transcript inside the nucleus. This processing involves a 3' non-translated region which adds polyadenylate nucleotides to the 3' end of the mRNA.

Transcription of DNA into mRNA is regulated by a region of DNA usually referred to as the "promoter". The promoter region contains a sequence of nucleotides which signals RNA polymerase to associate with the DNA, and initiate the production of a mRNA transcript using the DNA strand downstream from the promoter as a template to make a corresponding strand of RNA.

A number of promoters which are active in plant cells have been described in the literature. These include the nopaline synthase (NOS), octopine synthase (OCS) and mannopine synthase (MAS) promoters (which are carried on tumor-inducing plasmids of *Agrobacterium tumefaciens*), the cauliflower mosaic virus (CaMV) 19S and 35S promoters, and the light-inducible promoter from the small subunit of ribulose bis-phosphate carboxylase (ssRUBISCO, a very abundant plant polypeptide). These types of promoters have been used to create various types of DNA constructs which have been expressed in plants; see e.g., PCT publication WO 84/02913 (Rogers et al, Monsanto).

Promoters which are known or are found to cause production of a mRNA transcript in plant cells can be used in the present invention. The promoter selected should be capable of causing sufficient expression to result in the production of an effective amount of toxin protein to render the plant insecticidal to Lepidopteran larvae. Those skilled in the art recognize that the amount of toxin protein needed to induce the desired toxicity may vary with the particular Lepidopteran larvae to be protected against. Accordingly, while the CaMV35S and MAS promoters are preferred, it should be understood that these promoters may not be optimal promoters for all embodiments of the present invention.

The mRNA produced by the chimeric gene also contains a 5ʹ non-translated leader sequence. This sequence may be derived from the particular promoter selected such as the CaMV35S or MAS promoters. The 5ʹ non-translated region may also be obtained from other suitable eukaryotic genes or a synthetic gene sequence. Those skilled in the art recognize that the requisite functionality of the 5' non-translated leader sequence is the enhancement of the binding of the mRNA transcript to the ribosomes of the plant cell to enhance translation of the mRNA in production of the encoded polypeptide.

The chimeric gene also contains a structural coding sequence which encodes the toxic protein of *Bacillus thuringiensis.* It is known that Lepidopteran-type *B. thuringiensis* bacteria typically contains three toxin genes which have been classified on the basis of specific HindIII restriction fragments associated with each gene, see Kronstad et al. (1983). These genes are usually referred to as 4.5, 5.3 and 6.6 genes based on the size of the HindIII fragments obtained therefrom. Hence, for purposes of the present invention it should be understood that one can derive the coding sequence for an insecticidally-active toxin from one of the three genes. Hence, for purposes of the present invention by "toxin protein" is meant the full-length toxin as naturally produced by *Bacillus thuringiensis.*

Those skilled in the art will recognize that promoters useful in a particular embodiment will necessarily depend on the stability of the mRNA transcript.

The following examples will describe chimeric gene constructs employing the structural coding sequence of a "5.3 type" toxin gene from an isolate *B. thuringiensis* subspecies *kurstaki* HD-1. Those skilled in the art recognize that other subspecies of *Bacillus thuringiensis* exhibit toxicity toward the aforementioned Lepidopteran insects. These other subspecies include *B.t. kurstaki* HD-1 Dipel (PCT publication WO 86/01536), *B.t. sotto, B.t. berliner, B.t. thuringiensis, B.t. tolworthi, B.t. dendrolimus, B.t. alesti, B.t. galleriae, B.t. aizawai and B.t. subtoxicus.*

Isolation of DNA sequences encoding the toxin protein of *B. thuringiensis* is well known in the art. The coding sequence from the above-identified *B.t.* subspecies are quite homologous, particularly in the N-terminus region of the coding sequence. This homology is useful in the isolation of toxin protein coding sequences, since a DNA probe useful in the isolation of *B.t.* subspecies *kurstaki* HD-1 as described hereinafter would be useful in the isolation of toxin coding sequences from other subspecies. The amino acid sequence of the crystal protein toxin gene isolated from *Bacillus thuringiensis* subspecies *kurstaki* HD-1 was partially determined according to the method of Hunkapiller et al (1983). These sequences were verified using the DNA sequence of the NH₂-terminal portion of the crystal protein gene disclosed by Wong et al (1983). Synthetic oligo-nucleotide sequences based on an amino acid sequence determined from the crystal protein polypeptide were prepared according to the procedure of Beaucage et al (1981). The oligonucleotide probes prepared are as shown in Table I below.

**Table I**

| SYNTHETIC OLIGONUCLEOTIDE PROBES | | |
|---|---|---|
| Size | Probe Sequence | Area of B.t. Protein |
| 14-mer | TGG GGA CCG GAT TC | 1200 bp region |
| 14-mer | GAA AGA ATA GAA AC | * 27-31 amino acid region |
| 21-mer | CCT GAA GTA GAA-GTA TTA GGT | * 19-25 amino acid region |

| | | |
|---|---|---|
| * numbered from NH₂ - terminal end | | |

Plasmid DNA from *B. thuringiensis* subspecies *kurstaki* HD-1 was purified from 1 to 2 liters of culture according to the procedure of Kronstad et al (1983). All plasmid preparations were banded at least once in CsCl/ethidium bromide gradients. Plasmids 30 megadaltons and larger in size were preferentially isolated.

Digestion with restriction enzymes EcoRI, PstI, HindIII, BamHl and SmaI, was carried out according to conditions recommended by the supplied (Boehringer Mannheim). *Escherichia coli* strain JM 101 (Messing et al. 1981) and strain SR-200 were used as the recipients for the transformation step. Competent cells were prepared according to standard procedures (Dagert et al. 1979). Colonies transformed with plasmid pUC8, were plated on L-agar with 100 µg/ml of ampicillin and 40 µl of 4% 5-bromo-4-chloro-3-indolyl-β-D-galactopyranoside (x-gal).

Plasmid DNA was transferred to nitrocellulose according to the procedure of Southern (1975). Prehybridization was done by incubating the nitrocellulose paper with the bound transferred DNA in prehyridization fluid, 10 X Denhardt's (0.2% BSA, 0.2% Ficoll, 0.2% polyvinylpyrrolidone) and 6 X SSC (0.9M NaCl, 0.09M sodium citrate) for 2-4 hours at 37°C. Hybridization was done by incubating the nitrocellulose paper for 8-10 hours with 10-11 ml of the prehybridization fluid and the labelled probe. After several washes with 6X SSC at increasing temperatures (30-45°C) the paper was exposed to X-ray film.

BamHI-restricted pBR328 (100ng), treated with alkaline phosphatase (Boehringer Mannheim) was mixed and ligated with 500 ng of *B. thuringiensis* plasmid DNA restricted with BamHI. CaCl₂ prepared competent E. coli SR200 were transformed and selected by ampicillin resistance and screened for tetracycline sensitivity. Analysis by mini-plasmid prep procedures (Maniatis et al. 1982) identified two clones which had the correct 16 Kb insert. Southern hybridization analysis with radiolabelled probes from Table I demonstrated that the DNA fragment which contained the sequence hybridizing to the synthetic probe had been sub-cloned. The two plasmids, designated pMAP1 and pMAP2, differed only in the orientation of the DNA fragment within the vector. These plasmid constructs produced material cross-reactive to *B.t.* crystal protein toxin antibody when analyzed according to Western blot procedures (Geshoni et al. 1979). A restriction map of the inserted *B.t.* fragment was prepared and four EcoRI (E) sites and three Hind III (H) sites were located between the BamHI (B) sites. This is schematically illustrated as:

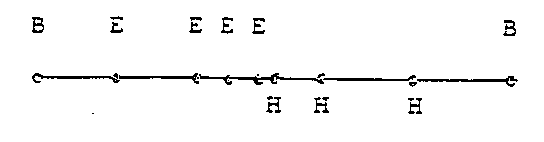
B E E E E B
H H H

*E. coli strain* SR200 containing pMAP2 has been deposited with the American Type Culture Collection, 12301 Parklawn Drive, Rockville, MD 20852 USA (hereinafter "ATCC") and has been designated ATCC accession number 39800.

An 8.1 Kb BamHI-PstI fragment was isolated after BamHI-PstI digestion of pMAP2 by electroelution from a preparative agarose gel onto DEAE paper used according to the directions of the manufacturer Schleicher & Schuell (see references). Plasmid pUC8 was used to sub-clone the BamHI-PstI fragment of pMAP2 carrying the *B.t.* gene. Ligation of pUC8 digested with BamHI and PstI with the purified 8.1 Kb BamHI-PstI fragment was followed by transformation of competent *E. coli* JM101. Transformants were selected on the basis of ampicillin resistance and a lack of β-galactosidase activity. A clone was isolated and was confirmed to contain the desired plasmid. This construct was designated pMAP3. *E. coli strain* JM101 containing pMAP3 has been deposited with ATCC and has been designated ATCC accession number 39801.

Reduction of the *B. thuringiensis* DNA insert of pMAP3 from 8.1 Kb to 4.6 Kb was accomplished by deleting a SmaI-HpaI fragment. Plasmid pMAP3 DNA, purified by CsCl gradient centrifugation was digested with SmaI and HpaI restriction enzymes and religated. The resulting DNA fragment was utilized to transform competent *E. coli* JM101 cells. Ampicillin resistant transformants were screened by agarose electrophoresis of mini-plasmid preparations. A clone was identified which contained a plasmid with the expected DNA restriction enzyme digestion pattern. This construct was designated pMAP4.

Lastly, the chimeric gene contains a 3ʹ non-translated region which encodes a polyadenylation signal which functions in plants to cause the addition of polyadenylate nucleotides to the 3ʹ end of the mRNA. Examples of suitable 3ʹ regions are the 3ʹ transcribed, non-translated regions containing the polyadenylated signal of the nopaline synthase (NOS) gene of the *Agrobacterium* tumor-inducing (Ti) plasmid or the conglycinin (7S) storage protein gene. An example of a preferred 3ʹ non-translated region is that from the NOS gene, described in the following examples.

The chimeric gene of the present invention is inserted into the genome of a tomato plant by any suitable method. Suitable plant transformation vectors include those derived from a Ti plasmid of *Agrobacterium tumefaciens* as well as those described in e.g. Herrera-Estrella 1983, Bevan 1983, Klee 1985 and EPO publication 120,516, Schilperoort et al. In addition to plant transformation vectors derived from the Ti or root-inducing (Ri) plasmids of *A. tumefaciens*, alternative methods can be used to insert the chimeric genes of this invention into tomato cells. Such methods may involve, for example, liposomes, electroporation and chemicals which increase free DNA uptake. Transformed cells are then cultured and regenerated into whole tomato plants.

### EXAMPLE 1

The insecticidal toxin gene from *Bacillus thuringiensis* subspecies *kurstaki* HD-1 has been previously described by Watrud et al. (1985). The toxin gene is contained on a 4.6 kb fragment of *B.t.* DNA in plasmid pMAP4. The DNA sequence of 3734 nucleotides of pMAP4, including the entire toxin protein coding sequence, was determined by the chain termination method of Sanger et al. (1977). The DNA sequence and the derived amino acid sequence for the toxin protein are shown in Figure 1. Nucleotide coordinates referred to hereinafter are based on the numbering of Figure 1.

This sequence includes 171 nucleotides upstream of the translational initiation codon and extends through a KpnI site 188 nucleotides after the translational termination codon. The first nucleotide of the protein coding sequence is labeled position +1. DNA sequences from nucleotide -75 to nucleotide 220 and from nucleotide 3245 to 3650 were also determined by the chemical method of Maxam and Gilbert (1977). The DNA sequence from -171 to -160 is from the known sequence of the plasmid vector pUC7 (Vieira, 1982) DNA sequence from -159 to -153 is from a chemically synthesized PstI linker (New England Biolabs); the three nucleotides from -152 to -150 are derived from the known cleavage site for restriction enzyme HpaI. The sequence from nucleotide -149 to -76 has been inferred from known 5'-flanking sequences of other *B.t.* toxin genes (Schnepf et al. 1985, Thorne et al. 1986, Adang et al. 1985 and Shibano et al. 1985).

### EXAMPLES 2-3

### Chimeric B.t. Toxin Genes for Plant Transformation with CaMV35S Promoter

### Full-Length Toxin

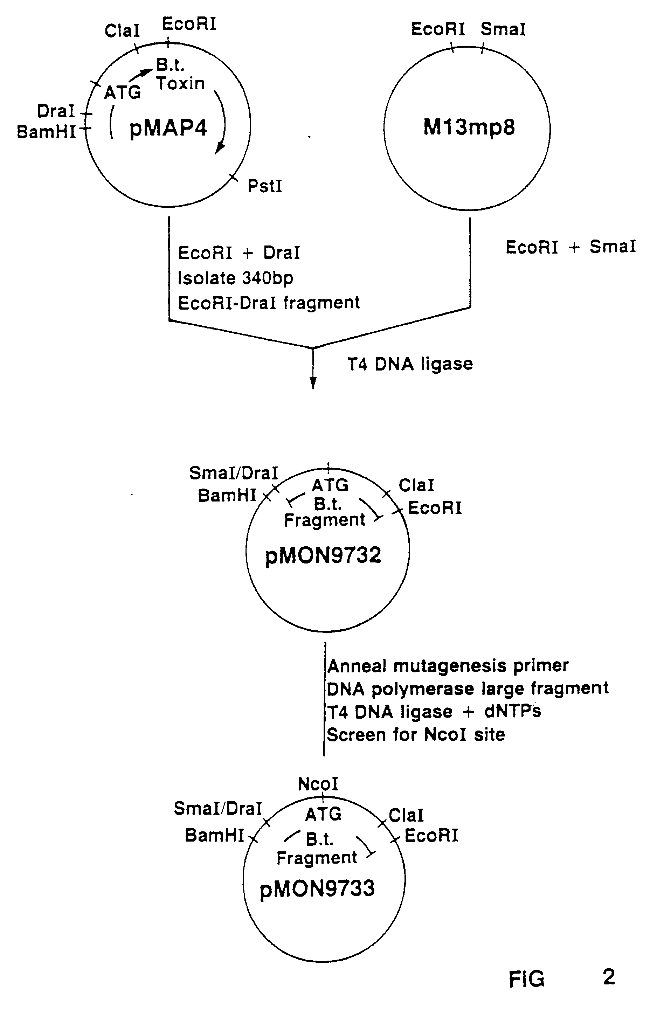

To make a chimeric gene encoding the toxin protein of *B.t.* a NcoI site is introduced at the translational initiation codon (ATG) of the DNA encoding the *B.t.* toxin such that the ATG codon is contained within the NcoI recognition site (CCATGG). DNA sequence analysis of the region of the toxin gene around the initiator codon revealed the sequence: 
5'-GAGATGGAGGTAACTTATGGATAACAATCCGA-3'
MetAspAsnAsnPro
 To introduce the desired NcoI site, it was necessary to change the sequence around the ATG from TTATGG to CCATGG. Referring to Figure 2, a 340 bp DraI-EcoRI fragment which includes the translational initiation region was subcloned from pMAP4 between the SmaI and EcoRI sites of the filamentous bacteriophage vector M13mp8. This plasmid was named pMON9732. Single-stranded phage DNA from this construct contains the noncoding strand of the toxin gene sequence.

Site-specific mutagenesis was performed on single-stranded DNA from this construct by the method of Zoller and Smith (1983) utilizing as a primer a synthetic oligonucleotide of the sequence:
5ʹ-GAGATGGAGGTAACCCATGGATAACAATCC-3ʹ
Following mutagenesis a clone containing the desired change was identified by digestion with NcoI, and the presence of the NcoI site was confirmed by DNA sequence analysis. This clone was designated pMON9733.

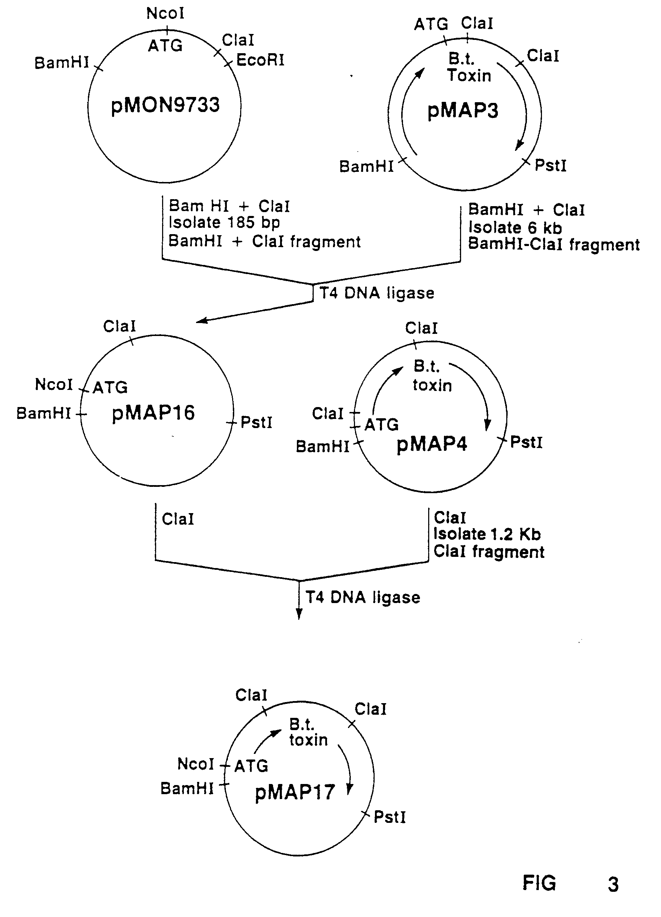

An intact toxin gene was constructed which incorporated the NcoI site from the site-specific mutagenesis described above. Referring to Figure 3, pMAP3 was digested with BamHI and ClaI and a fragment of containing the pUC8 vector and the toxin gene from the ClaI site at position 1283 to the PstI site beyond the end of the gene was isolated. A 185 bp fragment extending from the BamHI site in the mp8 multilinker to the ClaI site at position 106 was isolated from pMON9733. These two fragments were ligated to create pMAP16. pMAP16 contains the NcoI site at the ATG but is missing the segment of the toxin gene between the ClaI sites at 106 and 1283. This ClaI fragment was isolated from pMAP4 and ligated with ClaI digested pMAP16. A plasmid containing this inserted ClaI fragment in the proper orientation to reconstruct a functional toxin gene was identified and designated pMAP17. *E. coli* containing this plasmid produced a protein of about 134,000 daltons which reacted with antibodies prepared against purified crystal toxin protein from *Bacillus thuringiensis* subspecies kurstaki HD-1 at levels comparable to those produced by *E. coli* containing pMAP4. *E. coli* containing pMAP17 were toxic to the Lepidopteran larvae *Manduca sexta*.

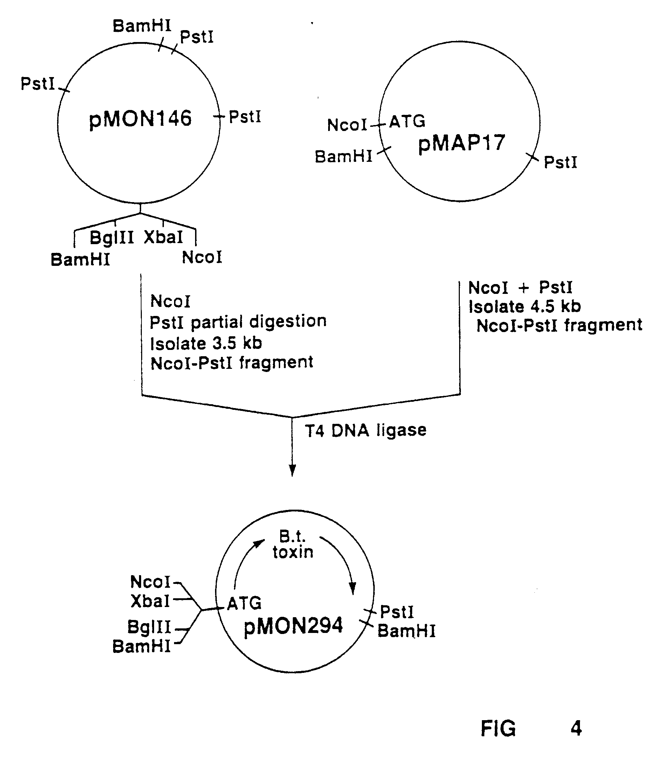

To facilitate construction of chimeric toxin genes in plant transformation vectors, BamHI and BglII sites were introduced just upstream of the NcoI site in the toxin gene. Referring to Figure 4, plasmid pMON146 was used as a source of a synthetic linker containing restriction sites for BamHI, BglII, XbaI and NcoI as shown: 
5'-GGATCCAGATCTGTTGTAAGGAGTCTAGACCATGGATC-3'
BamHI BglII XbaI NcoI
 pMON146 was partially digested with PstI and then digested to completion with NcoI, and a 3.5 kb NcoI-PstI fragment was isolated. The 4.5 kb NcoI-PstI fragment containing the entire toxin gene was isolated from pMAP17, and this fragment was ligated with the 3.5 kb pMON146 fragment. A plasmid containing these two fragments was designated pMON294. In pMON294 a BamHI and a BglII site are just upstream of the initiation codon for the toxin protein, and a BamHI site is just downstream of the PstI site.

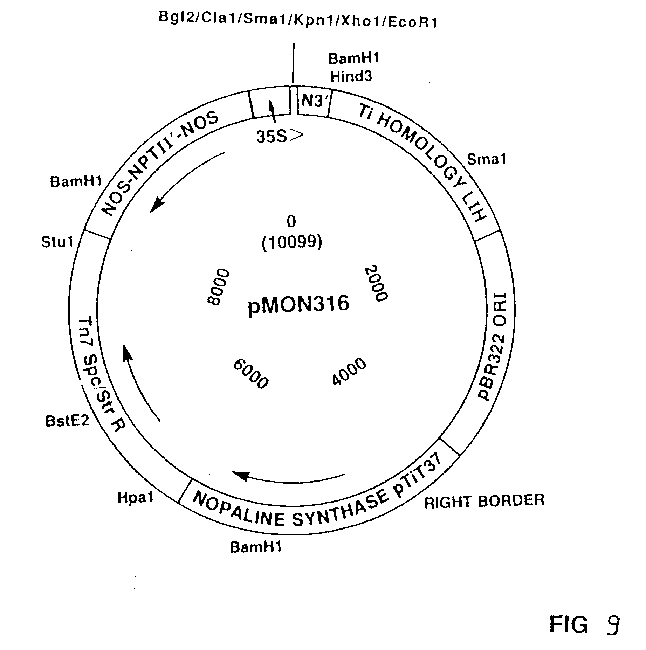

Referring to Figure 9, plasmid pMON316, a derivative of pMON200 (Fraley et al. 1985; Rogers et al, 1985) is a co-integrating type intermediate vector which contains the CaMV35S promoter and the 3' polyadenylation signal of the NOS gene. pMON316 has unique cleavage sites for the restriction endonucleases BglII, ClaI, KpnI, XhoI and EcoRI located between the 5' leader and the 3' NOS polyadenylation signals. The cleavage sites provide for the insertion of coding sequences carrying their own translational initiation signals immediately adjacent to the CaMV35S leader sequence. Plasmid pMON316 retains all the properties of pMON200 including spectinomycin resistance for selection in *E. coli and A. tumefaciens* as well as a chimeric kanamycin gene (NOS/NPTII/NOS) for selection of transformed plant tissue and the nopaline synthase gene for ready scoring of transformants and inheritance in progency.

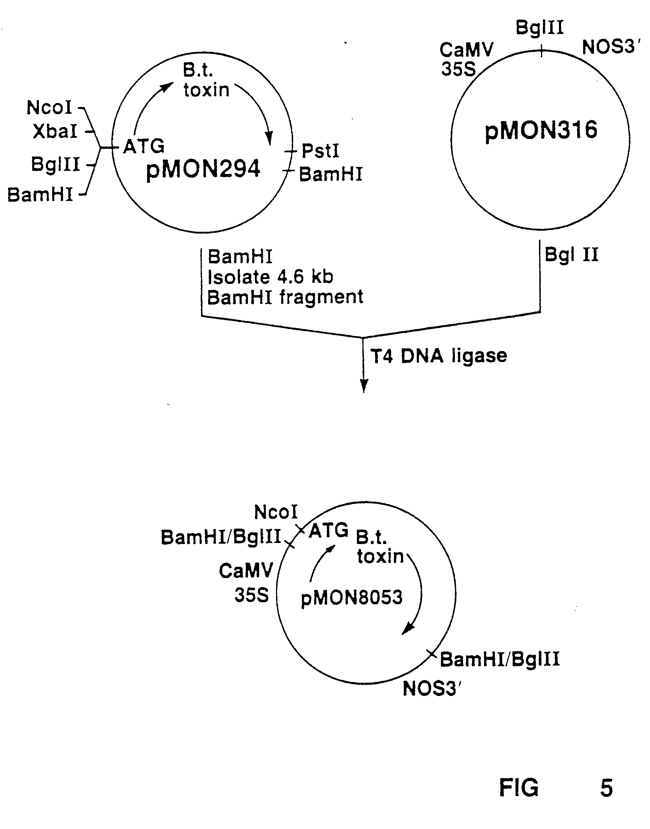

Referring to Figure 5, a plasmid was constructed for the expression of the *B.t.* toxin gene in plants by ligating the 4.5 kb BamHI fragment containing the toxin gene from pMON294 into pMON316 which had been digested with BglII. A plasmid which contained the toxin gene oriented such that the translational initiator was adjacent to the CaMV35S promoter was identified by digestion with EcoRI and designated pMON8053. Another chimeric plant gene was prepared comprising the full-length construct in which the structural coding sequence for the *B.t.* toxin was truncated at the DraI site at position 3479. This site is 10 nucleotides beyond the translational terminator codon for the coding sequence for the full-length *B.t* toxin. Thus, this construct contains the full-length coding sequence but very little 3' flanking sequence from the *B.t.* subspecies *kurstaki* gene. This construct is designated pMON9712.

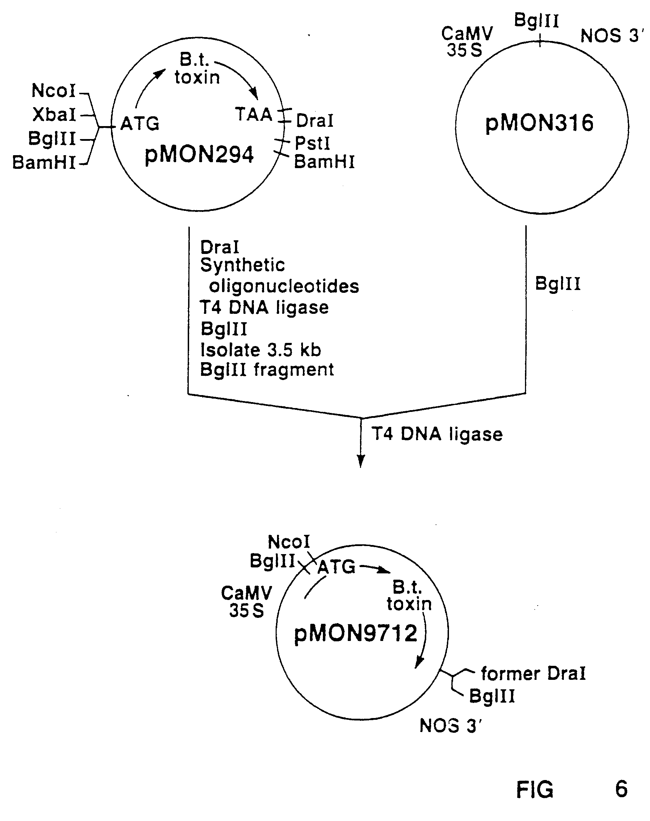

Referring to Figure 6, plasmid pMON9712 was prepared by digesting pMON294 with endonuclease DraI. A pair of complementary oligonucleotides having the following sequence were synthesized: 
5'-TAGTAGGTAGCTAGCCA-3'
3'-ATCATCCATCGATCGGTCTAG-5'
 When annealed to one another these oligonucleotides encode translational terminators in all three reading frames. The annealed oligonucleotide pair is flush-ended at one end and provides a four nucleotide single-stranded region capable of ligation to BglII digested DNA at the other end. The oligonucleotides were annealed to one another and ligated to pMON294 DNA which had been digested with DraI. The ligated DNA was digested with BglII, and a BglII fragment of approximately 3.5 kb containing the desired *B.t.* toxin coding sequence was isolated. This fragment extends from the BglII site just upstream of the translational initiation codon to the BglII site created by the oligonucleotide pair. This BglII fragment was ligated with BglII digested pMON316. A clone (pMON9712) was identified in which the translational intiator for the toxin gene was adjacent to the 35S promoter by digestion with EcoRI.

### EXAMPLE 4

### CHIMERIC B.t. TOXIN GENE FOR PLANT TRANSFORMATION USING MAS PROMOTER

A chimeric *B.t.* toxin gene was constructed in which the mannopine synthase (MAS) promoter from plasmid pTiA6, an octopine-type plasmid from *Agrobacterium tumefaciens* was used in combination with the *B.t.* toxin gene contained in previously described construct pMON9712.

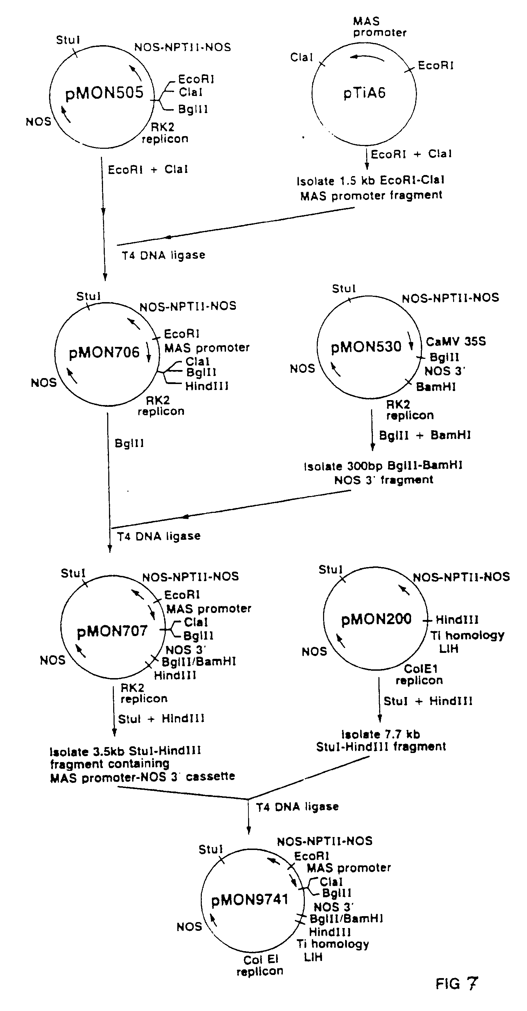

The MAS promoter was isolated from pTiA6 as a 1.5 kb EcoRI-ClaI fragment. This DNA fragment extends from the ClaI site at nucleotide 20,138 to the EcoRI site at 21,631 in the sequence of Barker et al. (1983). Referring to Figure 7, the EcoRI-ClaI fragment was ligated with the binary vector pMON505 (Horsch et al. 1986) which had been previously digested with EcoRI and ClaI. The resulting plasmid was designated pMON706. A fragment containing the NOS 3' end was inserted downstream of the MAS promoter to obtain a MAS-NOS 3' expression cassette vector. The NOS 3' fragment was excised from pMON530 as a 300 bp BglII-BamHI fragment and inserted into BglII-digested pMON706. The resulting plasmid was designated pMON707.

Plasmid pMON530 was constructed by cleavage of pMON200 with NdeI to remove a 900 bp NdeI fragment to create pMON503. Plasmid pMON503 was cleaved with HindIII and SmaI and mixed with plasmid pTJS75 (Schmidhauser and Helinski, 1985) that had also been cleaved with HindIII and SmaI. A plasmid that contained the 3.8 kb HindIII-SmaI fragment of pTJS75 joined to the 8 kb HindIII-SmaI fragment of pMON503 was isolated and designated pMON505. Next the CaMV35S-NOS3' cassette was transferred to pMON505 by cleavage of pMON316 with StuI and HindII and isolation of the 2.5 kb StuI-HindIII fragment containing the NOS-NPTII'-NOS marker and the CaMV35S-NOS3' cassette. This was added to pMON505 DNA cleaved with StuI and HindIII. Following ligation and transformation a plasmid carrying the CaMV35S-NOS3' cassette in pMON505 was isolated and designated pMON530.

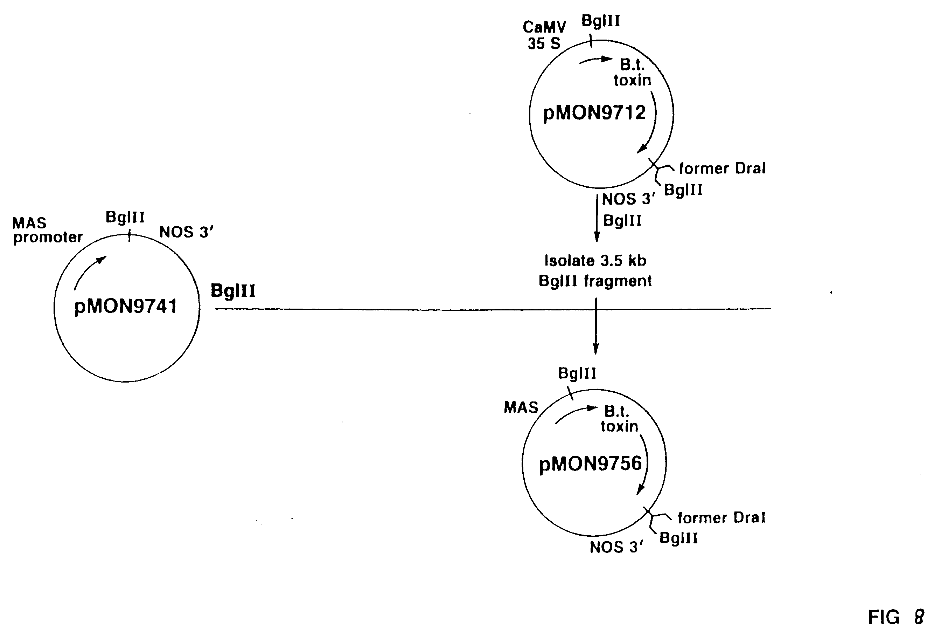

Since some binary vectors have greatly reduced frequencies of transformation in tomato as compared to co-integrating vectors, McCormick et al. (1986), the MAS-NOS 3' cassette was moved from pMON707 into the co-integrating vector pMON200, Fraley et al. (1985). Plasmid pMON200 was digested with StuI and HindIII and a 7.7 kb fragment isolated by agarose gel electrophoresis. Plasmid pMON707 was similarly digested with StuI and HindIII and a 3.5 kb StuI-HindIII fragment containing the MAS-NOS 3' cassette was isolated by agarose gel electrophoresis and recovery on a DEAE membranes with subsequent elution with 1M NaCl. These two DNA fragments were ligated and the resulting plasmid was designated pMON9741. This plasmid contains the MAS-NOS 3' cassette in the pMON200 co-integrating background. A chimeric *B.t.* toxin gene driven by the MAS promoter was prepared. Referring to Figure 8, the *B.t.* toxin gene insert was excised as a BglII fragment from pMON9712. This fragment was ligated with BglII-digested pMON9741. The 5' end of the *B.t.* toxin gene was adjacent to the MAS promoter by digestion with EcoRI. This plasmid was designated pMON9756 and contained the *B.t.* toxin coding sequence from pMON9712.

Plasmid pMON9756 can be used to transform tomato cells as described above. Tomato plants regenerated therefrom will exhibit useful toxicity toward susceptible Lepidopteran larvae.

### EXAMPLE 5

### EXPRESSION OF CHIMERIC B.t. GENES IN TOMATO PLANTS

### Introduction of Intermediate Vectors into Agrobacterium

Intermediate vectors containing full length toxin genes (pMON294, pMON8053 and pMON9712) were introduced into *Agrobacterium tumefaciens* strain GV311SE which contains the disarmed Ti plasmid pTiB6SE described by Fraley et al. (1985). *Agrobacterium tumefaciens* strains containing co-integrates between pTiB6SE and these intermediate vectors were selected as described.

### Transformation and Regeneration of Tomato

Transformation and regeneration of transformed tomato utilizing the *Agrobacterium tumefaciens* strains described above was performed as described by McCormick et al. (1986).

### Insect Feeding Assays of Transformed Tomato Plants

Toxicity of transformed tomato plants containing chimeric *B.t.* toxin genes was determined by feeding assays with test insects. In some experiments leaves were removed from plants and placed either singly or in groups of up to five leaves in sterile 82 mm Petri dishes containing moistened sterile filter paper. Five to ten insect larvae were applied to the leaves and allowed to feed for three to seven days at which time the larvae were scored for mortality and relative size. Alternatively, larvae were applied to the leaves of whole tomato plants growing in four inch pots which were enclosed in plastic boxes. Ripe tomato fruit were assayed for toxicity by applying insect larvae directly to the intact fruit. All assays were performed at room temperature.

*Manduca sexta* larvae used in feeding assays were obtained as eggs from Carolina Biological Supply Co., hatched at room temperature and applied to leaves or plants immediately after hatching.

### pMON9712 in Tomato

One tomato plant transformed using plant transformation vector pMON9712 was isolated as described above. Eleven F1 progeny from a self cross of this plant were recovered and assayed for toxicity to *Manduca sexta* neonatal larvae in two separate tests. The insect feeding assays were performed on whole plants as previously described.

Nine of the eleven progeny tested expressed nopaline synthase indicating the presence of the transforming DNA. Fewer than half of the larvae survived on eight of the nine nopaline positive plants. Fewer than twenty percent of the larvae survived on two of the nine nopaline positive plants. The survival rate for larvae on nontransformed plants was about ninety percent. These results indicate a significant level of toxicity in tomato plants transformed with pMON9712 as compared to nontransformed plants.

### REFERENCES:

Adang, M. J., Staver, M. J., Rocheleau, T.A., Leighton, J., Barker, R. F. and Thompson, D. V. (1985) Gene 26:289-300.

Baker, R. F., Idler, K. B., Thompson, D. V. and Kemp, J. D. (1983) Plant Mol. Biol. 2:335-350.

Beaucage, S. L. and Caruthers, M. H. (1981) Tetrahedron Lett 22:1859-1862; see also Addams, S. P. et al., (1983), JACS 105:661-663.

Bevan, M. et al. (1983), Nature 304:184.

Dagert, M. and Ehrlich, S. D. (1979) Gene 6:23-28.

Fraley, R. T., Rogers, S. G., Horsch, R. B., Eichholtz, D. A., Flick, J. S., Fink, C. L., Hoffmann, N. L. and Sanders, P. R. (1985) Bio/Technology 3:629-635.

Geshoni, J. M. and Palache, G. E. (1983) Anal. Biochem. 131:1-15, see also Towbin et al. (1979) PNAS-USA 76:4350-4354.

Herrera-Estrella, L., et al. (1983) Nature 303:209.

Horsch, R. B., Klee, H. (1986) PNAS-USA 83:4428-4432.

Hunkapiller, M. W., Hewick, R. M. Dreyer, W. H. and Hood, L. E., (1983) Methods in Enzymol. 91:399-413.

Klee, H. J., et al. (1985), Bio/Technology 3:637-42.

Kronstad, J. W., Schnepf, H. E. and Whiteley, H. R., (1983), J. Bacteriol. 154:419-428.

Maniatis, T., Fritsch, E. and Sambrook, J. (1982) Molecular Cloning - a Laboratory Manual Cold Spring Harbor, N. Y. 396 pp.

Maxam, A. M. and Gilbert W., (1977) Proc. Nat. Acad. Sci. U.S.A. 74:560-564.

McCormick, S., Niedermeyer, J., Fry, J., Barnason, A., Horsch, R. and Fraley, R. (1986) Plant Cell Rep. 5:81-84.

Messing, J., Crea, R. and Seeburg, P. H., (1981) Nucleic Acid Res. 9:309-321.

Rogers, S. G. et al. in Biotechnology in Plant Sciences, M. Zaitlin, P. Day and A. Hollaender, eds. Academic Press Orlando 214-226.

Rogers, S. G. et al. (1985) Plant Mol. Biol. Rptr. Vol 3:3:111-116.

Sanger, F. et al. (1977) Proc. Nat. Acad. Sci. U.S.A. 74:5463-5467.

Schleicher & Schuell, Inc., Keene, N. H. 03431 "Binding and Recovering of DNA and RNA using SIS NA-45 DEAE Membrane," Sequences - Application Update No 364.

Schmidhauser, T. J. and Helinski, D. R., (1985) J. Bacteriol. 164:155.

Schnepf, H. E., Wong, H. C. and Whiteley, H. R., (1985) J. Biol. Chem. 260:6264-6272.

Shibano, Y., Yamagata, A., Nakamura, N., Iizuka, T., Sugisaki, H. and Takanami, M. (1985) Gene 34:243-251.

Southern, E. M. (1975) J. Molec.. Biol. 98:503-517.

Thorne, L., Garduno, F., Thompson, T., Decker, D., Zounes, M., Wild, M., Walfeield, A. and Pollock, T., (1986) J. Bacteriol. 166:801-811.

Vieira, J. and Messing, J., (1982) Gene 19:259.

Watrud, L., Perlak, F., Tran, M., Kusano, K., Mayer, E., Miller-Wideman, M., Obukowicz, M., Nelson, D., Kreitinger, J. and Kaufman, R. (1985). Engineered Organisms in the Environment-Scientific Issues, ASM Press (Washington, D. C.).

Wong, H.C., Schnepf, H. E. and Whiteley, H. E. (1983) J. Biol. Chem. 258:1960-1967.

Zoller, M. J. and Smith, M. (1983) Methods in Enzymology 100:468-500.

## Claims

1. A method of producing genetically transformed tomato plants which exhibit insecticidal activity toward Lepidopteran larvae which comprises:
(a) inserting into the genome of a tomato cell a chimeric gene which comprises:
(i) a promoter which functions in plants to cause the production of a mRNA transcript;
(ii) a coding sequence that causes the production of mRNA encoding a toxin protein of Bacillus thuringiensis said mRNA being the transcript of the DNA sequence 1 to 3471 of Figure 1; and
(iii) a 3'non-translated region which functions in tomato to cause the addition of polyadenylate nucleotides to the 3' end of the mRNA;
(b) selecting transformed tomato cells; and
(c) regenerating from the transformed tomato cells genetically transformed tomato plants which exhibit insecticidal activity towards Lepidopteran larvae.

2. A method of Claim 1 in which the promoter is selected from the group consisting of the CaMV35S and MAS promoters.

3. A method of Claim 1 in which the promoter is the CaMV35S promoter.

4. A transformed tomato cell exhibiting insecticidal activity toward Lepidopteran larvae containing a chimeric gene comprising in sequence:
(a) a promoter which functions in plants to cause the production of a mRNA transcript;
(b) a coding sequence that causes the production of mRNA encoding a toxin protein of *Bacillus thuringiensis* said mRNA being the transcript of the DNA sequence 1 to 3471 of Figure 1; and
(c) a 3'non-translated region which functions in tomato to cause the addition of polyadenylate nucleotides to the 3' end of the mRNA.

5. A transformed tomato cell of Claim 4 in which the promoter is selected from the group consisting of CaMV35S and MAS promoters.

6. A cell of claim 4, in which the promoter is the CaMV35S promoter.

7. A differentiated tomato plant which exhibits insecticidal activity toward Lepidopteran larvae comprising transformed tomato cells of Claim 4.

8. A differentiated tomato plant of Claim 7 in which the promoter is selected from the group consisting of CaMV35S and MAS promoters.

9. A differentiated tomato plant of Claim 8 in which the promoter is the CaMV35S promoter.

10. The method of Claim 3 in which the protein toxin is from *Bacillus thuringiensis* subspecies *kurstaki* and the transformed plant exhibits insecticidal activity toward Lepidopterans of the genus *Manduca* and *Heliothis* and *Spodoptera exigua*.

11. The cell of Claim 6 in which the protein toxin is from *Bacillus thuringiensis* subspecies *kurstaki* and the transformed plant exhibits insecticidal activity toward Lepidopterans of the genus *Manduca* and *Heliothis* and *Spodoptera exigua*.

12. A differentiated tomato plant of Claim 8 in which the promoter is the CaMV35S promoter, the protein toxin is from *Bacillus thuringiensis* subspecies *kurstaki* and the transformed plant exhibits insecticidal activity toward Lepidopterans of the genus *Manduca* and *Heliothis* and *Spodoptera exigua*.

## Patentansprüche

1. Verfahren zur Herstellung genetisch transformierter Tomatenpflanzen, die eine insektizide Aktivität gegenüber Schmetterlingslarven aufweisen, welches Verfahren umfaßt:
(a) die Insertion eines chimären Gens in das Genom einer Tomatenzelle, welches chimäre Gen folgendes aufweist:
(i) einen Promotor, der in Pflanzen wirkt, um die Produktion eines mRNA-Transkripts zu bewirken;
(ii) eine Kodiersequenz, die die Erzeugung von mRNA bewirkt, welche für ein Toxinprotein von Bacillus thuringiensis kodiert, wobei diese mRNA das Transkript der DNA-Sequenz 1-3471 der Fig. 1 ist, und
(iii) einen 3'-Bereich, welcher nicht translatiert wird und in der Tomatenpflanze die Hinzufügung von Polyadenylatnukleotiden am 3'-Ende der mRNA bewirkt;
(b) die Selektion transformierter Tomatenzellen; und
(c) die Regeneration genetisch transformierter Tomatenpflanzen aus den transformierten Tomatenzellen, welche Tomatenpflanzen eine insektizide Aktivität gegenüber Schmetterlingslarven aufweisen.

2. Verfahren nach Anspruch 1, bei welchem der Promotor ausgewählt ist aus der Gruppe bestehend aus CaMV35S- und MAS-Promotoren.

3. Verfahren nach Anspruch 1, bei welchem der Promotor der CaMV35S-Promotor ist.

4. Transformierte Tomatenzelle, die eine insektizide Aktivität gegenüber Schmetterlingslarven aufweist und ein chimäres Gen enthält, welches nacheinander aufweist:
(a) einen Promotor, der in Pflanzen wirkt, um die Produktion eines mRNA-Transkripts zu bewirken;
(b) eine Kodiersequenz, die die Erzeugung von mRNA bewirkt, welche für ein Toxinprotein von Bacillus thuringiensis kodiert, wobei diese mRNA ein Transkript der DNA-Sequenz 1-3471 der Fig. 1 ist, und
(c) einen 3'-Bereich, welcher nicht translatiert wird und in der Tomatenpflanze die Hinzufügung von Polyadenylatnukleotiden an das 3'-Ende der mRNA bewirkt.

5. Transformierte Tomatenzelle nach Anspruch 4, bei welcher der Promotor ausgewählt ist aus der Gruppe bestehend aus CaMV35S- und MAS-Promotoren.

6. Zelle nach Anspruch 4, bei welcher der Promotor der CaMV35S-Promotor ist.

7. Differenzierte Tomatenpflanze, die eine insektizide Aktivität gegenüber Schmetterlingslarven aufweist und transformierte Tomatenzellen nach Anspruch 4 umfaßt.

8. Differenzierte Tomatenpflanze nach Anspruch 7, bei welcher der Promotor ausgewählt ist aus der Gruppe bestehend aus CaMV35S- und MAS-Promotoren.

9. Differenzierte Tomatenpflanze nach Anspruch 8, bei welcher der Promotor der CaMV35S-Promotor ist.

10. Verfahren nach Anspruch 3, bei welchem das Proteintoxin von Bacillus thuringiensis, Subspezies kurstaki stammt und die transformierte Pflanze eine insektizide Aktivität gegenüber Schmetterlingen der Gattung Manduca und Heliothis und Spodoptera exigua aufweist.

11. Zelle nach Anspruch 6, bei welcher das Proteintoxin von Bacillus thuringiensis, Subspezies kurstaki stammt und die transformierte Pflanze eine insektizide Aktivität gegenüber Schmetterlingen der Gattung Manduca und Heliothis und Spodoptera exigua aufweist.

12. Differenzierte Tomatenpflanze nach Anspruch 8, bei welcher der Promotor der CaMV35S-Promotor ist, das Proteintoxin von Bacillus thuringiensis, Subspezies kurstaki stammt und die transformierte Pflanze eine insektizide Aktivität gegenüber Schmetterlingen der Gattung Manduca und Heliothis und Spodoptera exigua aufweist.

## Revendications

1. Procédé pour produire des plantes de tomates génétiquement transformées qui présentent une activité insecticide vis-à-vis de larves de lépidoptères, qui comprend le opérations consistant à :
(a) Insérer dans le génome d'une cellule de tomate un gène chimère qui comprend :
(i) un promoteur qui fonctionne dans les plantes pour provoquer la production d'ARN-m de transcription ;
(ii) une séquence codante qui provoque la production d'ARN-m codant pour une toxine protéique de *Bacillus thuringiensis*, ledit ARN-m étant le produit de transcription de la séquence d'ADN 1 à 3471 de la figure 1 ; et
(iii) une région 3' non traduite qui fonctionne dans la tomate pour provoquer l'addition de polynucléotides polyadénylates à l'extrémité 3' de l'ARN-m ;
(b) sélectionner les cellules de tomates transformées ; et
(c) régénérer à partir des cellules de tomates transformées, des plantes de tomates génétiquement transformées qui possèdent une activité insecticide vis-à-vis des larves de lépidoptères.

2. Procédé selon la revendication 1, dans lequel le promoteur est choisi dans le groupe constitué par les promoteurs CaMV35S et MAS.

3. Procédé selon la revendication 1, dans lequel le promoteur est le promoteur CaMV35S.

4. Cellule de tomate transformée présentant une activité insecticide vis-à-vis de larves de lépidoptères, contenant un gène chimère comprenant en séquence :
(a) un promoteur qui fonctionne dans les plantes pour provoquer la production d'un ARN-m de transcription ;
(b) une séquence codante qui provoque la production d'ARN-m codant pour une toxine protéique de *Bacillus thuringiensis*, ledit ARN-m étant le produit de transcription de la séquence d'ADN 1 à 3471 de la figure 1 ; et
(c) une région 3' non traduite qui fonctionne dans la tomate pour provoquer l'addition de polynucléotides polyadénylates à l'extrémité 3' de l'ARN-m ;

5. Cellule de tomate transformée selon la revendication 4, dans laquelle le promoteur est choisi dans le groupe constitué par les promoteurs CaMV35S et MAS.

6. Cellule selon la revendication 4, dans laquelle le promoteur est le promoteur CaMV35S.

7. Plante de tomate différenciée qui présente une activité insecticide vis-à-vis des larves de lépidoptères, comprenant des cellules de tomate transformées selon la revendication 4.

8. Plante de tomate différenciée selon la revendication 7, dans laquelle le promoteur est choisi dans le groupe constitué par les promoteurs CaMV35S et MAS.

9. Plante de tomate différenciée selon la revendication 8, dans laquelle le promoteur est le promoteur CaMV35S.

10. Procédé selon la revendication 3, dans lequel la toxine protéique provient de *Bacillus thuringiensis*, sous-espèce *kurstaki* et la plante transformée présente une activité insecticide vis-à-vis des lépidoptères du genre *Manduca* et *Heliothis* et vis-à-vis de *Spodoptera exigua*.

11. Procédé selon la revendication 6, dans lequel la toxine protéique provient de *Bacillus thuringiensis*, sous-espèce *kurstaki* et la plante transformée présente une activité insecticide vis-à-vis des lépidoptères du genre *Manduca* et *Heliothis* et vis-à-vis de *Spadoptera exigua*.

12. Plante de tomate différenciée selon la revendication 8, dans laquelle le promoteur est te promoteur CaMV35S, la toxine protéique provient de *Bacillus thuringiensis*, sous-espèce *kurstaki* et la plante transformée présente une activité insecticide vis-à-vis des lépidoptères du genre *Manduca* et *Heliothis* et vis-à-vis de *Spodaptera exigua*.
